# EUROPEAN PATENT SPECIFICATION

(11) **EP 2 813 232 B1**
(45) Date of publication and mention of the grant of the patent: **09.08.2017**
(21) Application number: 13171495.8
(22) Date of filing: 11.06.2013
(51) Int. Cl.: A61K 35/16, A61K 35/19, A61L 27/54, A61K 45/06, A61P 17/02

(54) **Process of Production of Allogenic Growth Factor Extract**
Verfahren zur Herstellung eines allogenen Wachstumsfaktorextrakts
Procédé de production d'extrait de facteur de croissance allogène

(43) Date of publication of application: 17.12.2014
(73) Proprietor: DOT GmbH, 18059 Rostock (DE)
(72) Inventor: Neumann, Hans-Georg, 18146 Rostock (DE); Neumann, Barbara., 18146 Rostock (DE)
(74) Representative: Prinz & Partner mbB

(56) References cited:
- WO-A1-97/34614
- WO-A1-2006/137778
- WO-A2-01/60424
- US-A- 4 479 896
- US-A1- 2012 156 306
- KSANDER G A ET AL: "The effect of platelet releasate on wound healing in animal models", JOURNAL OF THE AMERICAN ACADEMY OF DERMATOLOGY, C.V. MOSBY, ST. LOUIS, MO, US, vol. 22, no. 5, 1 May 1990 (1990-05-01), pages 781-791, XP025650489, ISSN: 0190-9622, DOI: 10.1016/0190-9622(90)70109-U [retrieved on 1990-05-01]
- SCHALLMOSER KATHARINA ET AL: "Preparation of pooled human platelet lysate (pHPL) as an efficient supplement for animal serum-free human stem cell cultures", JOVE, JOURNAL OF VISUALIZED EXPERIMENTS, JOURNAL OF VISUALIZED EXPERIMENTS, US, no. 32, 1 January 2009 (2009-01-01), XP009148870, ISSN: 1940-087X, DOI: DOI:10.3791/1523
- Natalie Fekete ET AL: "Platelet lysate from whole blood-derived pooled platelet concentrates and apheresis-derived platelet concentrates for the isolation and expansion of human bone marrow mesenchymal stromal cells: production process, content and identification of active components", Cytotherapy, vol. 14, no. 5, 1 May 2012 (2012-05-01), pages 540-554, XP055178087, ISSN: 1465-3249, DOI: 10.3109/14653249.2012.655420

## Description

### Technical Field:

The present invention relates to a process for producing an allogenic growth factor extract.

### Background art:

At present the gold standard for treating extensive soft tissue defects, typically in connection with cancer diseases, relates to implantation of autogenic bone material taken from the hip bone of the patient. The patient, however, is due to the autogenic bone transfer faced with additional risks, such as an infection risk, and strains, such as increased pain sensation at the area of bone removal.

To overcome such drawbacks, alloplastic and allogenic materials have been developed, which presently do not seem to be practical in clinical use due to rejection problems based on immunologic reactions concerning the alloplastic and allogenic materials in the patient.

Alternatively, it is suggested to transfer autogenic fatty tissue. However, this treatment does also not seem to be practical in clinical use, as volume reductions of the transferred fatty tissue have been observed in long term studies.

To improve wound healing and, thus, clinical success of implants, it is generally discussed to use autogenic growth factors in particular as coating materials for respective implants. However, the use of autogenic growth factors is limited in particular in case the patient does not produce enough growth factors himself and/or the time span until use is too short.

To overcome the drawbacks there are attempts to use xenogenic growth factors, which are taken from pig bladder. However, the use of xenogenic growth factors is presently only allowed in the United States of America.

Thus, there is a need for providing an alternative solution of improving clinical success of wound healing, in particular of wound healing in relation with implants. Furthermore, there is a need for providing a simple, time and cost effective production process for obtaining growth factor extracts suitable for all patients independent of e.g. the blood group, and provided in stabilized form to facilitate storage until use.

### Brief Description of the Invention:

The aforementioned needs are met in part or all by means of the claimed inventive subject matter. Preferred embodiments are in particular described in the dependent claims and the detailed description.

Accordingly, the present invention refers to a process for producing an allogenic growth factor extract, characterized in that the process comprises or consists of the following steps:
a. providing human plasma comprising one or more platelets and plasma proteins,
b. separating at least part of the platelets by suitable means from the plasma and the plasma proteins,
c. admixing at least part of the separated platelets from step b) with a suitable amount of water to release one or more, the same or different growth factors from the platelets and separating at least part of the released growth factors by suitable means from the residue to provide an allogenic growth factor extract, and
d. freeze-drying at least part of the separated allogenic growth factor extract in step c).

The aforementioned inventive embodiments can - as far as reasonable in view of a technical expert - comprise any possible combination of the preferred embodiments, which are disclosed in the following and in particular in the dependent claims.

### Detailed Disclosure of the Invention:

The inventors provide a simple, time and cost effective inventive production process for obtaining a stabilized allogenic growth factor extract starting from human plasma. Due to the mild production conditions of the inventive multiple-step process, the obtainable growth factors are intact, can be stored up to 24 to 36 months in freeze-dried condition and can be used for all patients independent of e.g. the blood group, as potentially interacting proteins are separated from the extract in particular during the washing step. The concentration of the growth factors can in addition be easily adjusted by addition of suitable solvent to the inventively stabilized freeze-dried allogenic growth factor extract.

Furthermore, the inventive production step d) of freeze-drying the separated allogenic growth factor extract facilitates - in contrast to other suitable stabilizing methods, such as sterile filtration - sterilization using gamma radiation, which in turn increases stabilization of the obtained allogenic growth factor extract.

In connection with the present invention the term "human plasma" is derived from human whole blood that is preferably anticoagulated with citrate dextrose. Human plasma generally comprises platelets and plasma proteins and more preferably is essentially free of red blood cells. Typically the platelet count in the plasma is about 150 000 platelets / µl or more. Preferably a "human platelet-rich plasma" (human PRP) is used as "human plasma" in the context of the present invention, wherein the platelet count is preferably ≥ 350 000 platelets / µl, more preferably ≥ 500 000 platelets / µl, even more preferably ≥ 750 000 platelets / µl, further more preferably ≥ 1 000 000 platelets / µl.

In a preferred embodiment, the human plasma, preferably human PRP to be used in step a) of the inventive process has not been treated prior to use in step a) in such a way that at least part of the platelets, preferably essentially all platelets have released part or essentially all of the growth factors into the plasma. Thus, the human plasma, preferably human PRP has preferably not be stored under those conditions, e.g. multiple times freezing or warming to temperatures > 37 °C prior to use in step a) of the inventive process, as in this case some growth factors, e.g. TGF- β, may be damaged.

Accordingly, in a further preferred embodiment the human plasma, preferably human PRP is stored under suitable agitation, even more preferably stored under suitable agitation within a temperature range of 4 °C to 25 °C, more preferably 20 °C to 22 °C prior to inventive use. In addition it is further preferred to store the human plasma, preferably human PRP as little as possible and use it inventively as prompt as possible to achieve a good yield of growth factors. As an example, human plasma, preferably human PRP should be used prior to 5 days storage at 22 °C or prior to 14 days storage at 4 °C.

In connection with the present invention the term "plasma protein" generally refers to proteins directly present in the liquid plasma. In a preferred embodiment of the present invention the term "plasma protein" preferably refers to proteins, which do not stimulate wound healing and which potentially interact with the immunologic defense of a recipient patient. More preferably the plasma protein is selected from one or more different proteins of the group consisting of globulines, preferably immunoglobulines (antibodies), more preferably of antibodies directed against blood groups.

According to the inventive process step b) at least part of the platelets, preferably essentially all platelets are separated by suitable means from the plasma and the plasma proteins. In a preferred embodiment at least part of the platelets, preferably all platelets are in a first step separated from plasma by suitable means, preferably by centrifugation, filtration, sedimentation, and/or decantation. According to a preferred embodiment the plasma is centrifuged for 10 to 20 minutes, preferably 15 minutes, at 3000 to 6000, preferably 4000 revolution per minute (RPM), and 2 °C to 6 °C, preferably 4 °C.

In a second step of process step b) of the inventive process at least part of the platelets, preferably essentially all platelets are essentially separated from potentially remaining plasma protein, preferably by washing at least part of the separated platelets one or more time with a suitable washing agent, preferably selected from suitable blood cell compatible buffer solutions, more preferably saline solution (preferably 0.9 wt.-% NaCl), phosphate buffered saline solution (PBS), ringer's solution, simulated body fluid solution (SBF), most preferably 0.9 wt.-% NaCl saline solution. The amount of suitable washing agent should be kept as little as possible to reduce the application of energy and, thus, possible damage of the platelets. E.g., per human plasma, preferably human PRP with 200 to 300 ml the amount of washing agent should preferably be at least 100 ml to maximum 300 ml per washing step. At least part or preferably essentially all washed platelets are separated from the washing agent by suitable means, preferably by centrifugation, filtration, sedimentation, and/or decantation. According to a preferred embodiment the plasma is centrifuged for 10 to 20 minutes, preferably 15 minutes, at 3000 to 6000, preferably 4000 RPM, and 2 °C to 6 °C, preferably 4 °C.

According to step c) of the inventive process at least part of the separated platelets, preferably essentially all separated platelets from step b) are admixed in a first step with a suitable amount of water to release one or more, the same or different growth factors from the platelets. In a preferred embodiment the water is selected from distilled water or deionized water. According to a further preferred embodiment the amount of water in step c) is kept as little as possible, to reduce the time of the freeze-drying step d).

Subsequently, at least part, preferably essentially all of the released growth factors are separated by suitable means in a second step of process step c) of the inventive process from the residue to provide an allogenic growth factor extract. Preferably at least part or preferably essentially all released growth factors are separated from the residue by centrifugation, filtration, sedimentation or decantation. According to a further preferred embodiment the aqueous mixture of released growth factors and residue is centrifuged for 10 to 20 minutes, preferably 15 minutes, at 3000 to 6000, preferably 4000 RPM, and 2 °C to 6 °C, preferably 4 °C.

According to a preferred embodiment of the present invention process step c) is repeated one, two, three or more times to increase the yield of growth factors and the growth factors separated in each step are combined to form the allogenic growth factor extract in step c).

According to process step d) of the inventive process at least part of the separated allogenic growth factor extract from step c) is freeze-dried to facilitate storage of the inventive allogenic growth factor extract.

The inventive process is furthermore advantageous, as freeze-drying facilitates sterilization of the stabilized separated growth factor extract in particular by use of gamma radiation, which is a cost effective sterilization method and allows that the inventive process steps can be carried out under low-germ conditions and, thus, does not require the use of cost intensive sterile conditions.

In accordance with another preferred embodiment of the freeze-drying step d) of the inventive process, suitable freezing and drying methods are applied.

Preferably, the separated allogenic growth factor extract is - depending on the amount of solution - in a first step frozen, preferably for 8 to 24 hours at temperatures between -30 °C to -20 °C, and in a second step dried for 12 to 32 hours at temperatures between 20 °C to 30 °C to form the freeze-dried allogenic growth factor extract.

To allow batch formation the obtained freeze-dried allogenic growth factor extract, at least part of the growth factors, preferably essentially all growth factors are determined and the allogenic growth factor extract is preferably labeled accordingly.

The aforementioned inventive embodiments can - as far as reasonable in view of a technical expert - comprise any possible combination of the preferred embodiments, which are disclosed hereinbefore.

The allogenic growth factor extract obtained according to the above production process can be used for at least partially coating implants or soft tissue wound healing material.

The implant or the soft tissue wound healing material can be coated by any suitable means, preferably by spray coating, dip coating, etc. of a resolubilized growth factor extract.

The implant which is at least partially coated with the allogenic growth factor is preferably an implant having direct contact with bone material of the patient, wherein the coating with the growth factor extract is preferably only present in the area of bone contact. More preferably the suitable implants are selected from the group consisting of spinal cages implants, knee implants, hip implants.

The soft tissue wound healing material which is at least partially coated with the allogenic growth factor implant is preferably a material directly in contact with the wounded soft tissue, more preferably selected from the group consisting of mull or non-woven materials, preferably compresses or patches.

The present invention is described in the following on the basis of exemplary embodiments, which merely serve as examples and which shall not limit the scope of the present protective right.

### Executive examples:

### A) Production of an inventive allogenic growth factor extract

### Inventive process step a)

Providing a transfusion bag comprising human PRP having 2 to 4x10¹¹ platelets per 200 to 300 ml human PRP.

### Inventive process step b)

Transfering the PRP to a suitable vial for centrifugation and conducting centrifugation for 15 minutes at 4000 RPM and 4 °C. The sediment comprises the separated platelets and is separated from the supernatant for further preparation.

Subsequently, the separated platelets are washed with 300 ml saline solution (0.9 wt.-% NaCl in water) in a suitable vial. The thus prepared washing suspension is provided on a plate shaker for 15 minutes at 180 RPM. Subsequently the vial is centrifuged for 15 minutes at 4000 RPM and 4 °C. The sediment comprises the separated platelets and is separated from the supernatant for further preparation.

### Inventive process step c)

Subsequently, the washed and separated platelets are admixed with 120 ml distilled water in order to lyse the platelets. The thus prepared admixture is provided on a plate shaker for 15 minutes at 180 RPM. Subsequently the vial is centrifuged for 15 minutes at 4000 RPM and 4 °C. The supernatant comprises the allogenic growth factor extract and is separated from the sediment for further preparation.

The inventive process step c) is repeated twice.

### Inventive process step d)

Transferring the separated supernatant into a suitable vial for freeze drying.

Freezing the supernatant comprising the allogenic growth factor extract at least at - 20 °C at steady rotation and drying the frozen supernatant with suitable means.

The inventively freeze-dried allogenic growth factor extract is transferred to a suitable sealable vial. Dependent on the starting PRP material the yield of the allogenic growth factor extract lies in the range of 200 to 300 mg.

In order to form batches, the growth factors of the inventive allogenic growth factor extract are determined in a further step and the package is labeled accordingly.

The individual features of the executive example can independently from each other be combined with preferred features of the general description.

## Claims

1. Process for producing an allogenic growth factor extract, **characterized in that** the process comprises or consists of the following steps:
a. providing human plasma comprising one or more platelets and plasma proteins,
b. separating at least part of the platelets by suitable means from the plasma and the plasma proteins,
c. admixing at least part of the separated platelets from step b) with a suitable amount of water to release one or more, the same or different growth factors from the platelets and separating at least part of the released growth factors by suitable means from the residue to provide an allogenic growth factor extract, and
d. freeze-drying at least part of the separated allogenic growth factor extract.

2. Process according to claim 1, wherein the human plasma has not been treated prior to use in step a) in such a way that at least part of the platelets, preferably essentially all platelets have released part or essentially all of the growth factors into the plasma.

3. Process according to claim 1 or 2, wherein in step b) the platelets are in a first step separated from the plasma by centrifugation and in a second step at least part of the separated platelets are subsequently washed one or more times with a suitable washing agent to separate the platelets from the plasma proteins, preferably wherein the suitable washing agent is selected from the group consisting of saline solution, phosphate buffered saline solution (PBS), Ringer's solution, simulated body fluid solution (SBF), preferably 0.9 wt.-% NaCl saline solution.

4. Process according to any one of claims 1 to 3, wherein the plasma proteins in step a) and step b) comprise or consist of one or more proteins selected from the group consisting of globulines, preferably immunoglobulines, more preferably antibodies against blood groups.

5. Process according to any one of claims 1 to 4, wherein the washing process in step c) is repeated one, two, three or more times and the growth factors separated in each separation step are combined to form the allogenic growth factor extract in step c).

6. Process according to any one of claims 1 to 5, wherein distilled water or deionized water, preferably distilled water is used in step c) as the water.

7. Process according to any one of claims 1 to 6, wherein in step d) the separated allogenic growth factor extract is in a first step frozen, preferably for 8 to 24 hours at temperatures between -30 °C to -20 °C, and in a second step dried for 12 to 32 hours at temperatures between 20 °C to 30 °C to form the freeze-dried allogenic growth factor extract.

8. Process according to any one of claims 1 to 7, wherein in a further process step at least part of the growth factors in the freeze-dried allogenic growth factor extract are determined and the allogenic growth factor extract is labeled accordingly.

## Patentansprüche

1. Verfahren zur Herstellung eines allogenen Wachstumsfaktorextrakts, **dadurch gekennzeichnet, dass** das Verfahren die folgenden Schritte umfasst oder aus den folgenden Schritten besteht:
a. Bereitstellen von menschlichem Plasma mit einem oder mehreren Blutplättchen und Plasmaproteinen,
b. Abtrennen zumindest eines Teils der Blutplättchen aus dem Plasma und den Plasmaproteinen mit geeigneten Mitteln,
c. Vermischen zumindest eines Teils der abgetrennten Blutplättchen aus Schritt b) mit einer geeigneten Menge Wasser, um einen oder mehrere identische oder unterschiedliche Wachstumsfaktoren aus den Blutplättchen freizusetzen, und Abtrennen zumindest eines Teils der freigesetzten Wachstumsfaktoren aus dem Rückstand mit geeigneten Mitteln, um einen allogenen Wachstumsfaktorextrakt bereitzustellen, und
d. Gefriertrocknen zumindest eines Teils des abgetrennten allogenen Wachstumsfaktorextrakts.

2. Verfahren nach Anspruch 1, bei dem das menschliche Plasma vor der Verwendung in Schritt a) nicht derart behandelt wurde, dass zumindest ein Teil der Blutplättchen, vorzugsweise im Wesentlichen alle Blutplättchen einen Teil der Wachstumsfaktoren oder im Wesentlichen alle Wachstumsfaktoren in das Plasma freigesetzt haben.

3. Verfahren nach Anspruch 1 oder 2, bei dem in Schritt b) die Blutplättchen in einem ersten Schritt durch Zentrifugieren aus dem Plasma abgetrennt werden und zumindest ein Teil der abgetrennten Blutplättchen dann in einem zweiten Schritt einmal oder mehrere Male mit einem geeigneten Waschhilfsmittel gewaschen werden, so dass die Blutplättchen aus den Plasmaproteinen abgetrennt werden, wobei das geeignete Waschhilfsmittel vorzugsweise aus der Gruppe ausgewählt ist, die aus einer Kochsalzlösung, einer phosphatgepufferten Salzlösung (PBS), einer Ringerlösung, einer Lösung aus simulierter Körperflüssigkeit (SBF), vorzugsweise einer NaCl-Lösung mit 0,9 Gew.-% besteht.

4. Verfahren nach einem der Ansprüche 1 bis 3, bei dem die Plasmaproteine in Schritt a) und Schritt b) ein oder mehrere Proteine umfassen oder daraus bestehen, die aus Gruppe ausgewählt sind, die aus Globulinen, vorzugsweise Immunglobulinen und weiter bevorzugt aus Antikörpern gegen Blutgruppen besteht.

5. Verfahren nach einem der Ansprüche 1 bis 4, bei dem der Waschvorgang in Schritt c) einmal, zweimal, dreimal oder öfter wiederholt wird und die in jedem Abtrennschritt abgetrennten Wachstumsfaktoren so kombiniert werden, dass der allogene Wachstumsfaktorextrakt in Schritt c) gebildet wird.

6. Verfahren nach einem der Ansprüche 1 bis 5, bei dem in Schritt c) destilliertes Wasser oder deionisiertes Wasser, vorzugsweise destilliertes Wasser als Wasser verwendet wird.

7. Verfahren nach einem der Ansprüche 1 bis 6, bei dem in Schritt d) der abgetrennte allogene Wachstumsfaktorextrakt in einem ersten Schritt vorzugsweise für eine Dauer von 8 bis 24 Stunden bei Temperaturen zwischen -30°C und -20°C tiefgefroren und in einem zweiten Schritt für eine Dauer von 12 bis 32 Stunden bei Temperaturen zwischen 20°C und 30°C getrocknet wird, so dass der gefriergetrocknete allogene Wachstumsfaktorextrakt gebildet wird.

8. Verfahren nach einem der Ansprüche 1 bis 7, bei dem in einem weiteren Verfahrensschritt zumindest ein Teil der Wachstumsfaktoren in dem gefriergetrockneten allogenen Wachstumsfaktorextrakt bestimmt wird und der allogene Wachstumsfaktorextrakt dementsprechend gekennzeichnet wird.

## Revendications

1. Procédé de fabrication d'un extrait de facteur de croissance allogénique, **caractérisé en ce que** le procédé comprend ou se compose des étapes suivantes :
a. fourniture de plasma humain comprenant une ou plusieurs plaquettes sanguines et protéines plasmatiques,
b. séparation d'au moins une partie des plaquettes sanguines du plasma et des protéines plasmatiques par des moyens appropriés,
c. mélange d'au moins une partie des plaquettes sanguines séparées de l'étape b) avec une quantité d'eau appropriée pour libérer un ou plusieurs facteurs de croissance identiques ou différents des plaquettes sanguines, et séparation d'au moins une partie des facteurs de croissance libérés du résidu avec des moyens appropriés pour fournir un extrait de facteur de croissance allogénique, et
d. lyophilisation d'au moins une partie de l'extrait de facteur de croissance allogénique séparé.

2. Procédé selon la revendication 1, dans lequel le plasma humain n'a pas été traité avant l'utilisation à l'étape a) de sorte qu'au moins une partie des plaquettes sanguines, de préférence sensiblement toutes des plaquettes sanguines ont libéré une partie ou sensiblement l'ensemble des facteurs de croissance dans le plasma.

3. Procédé selon la revendication 1 ou 2, dans lequel à l'étape b), les plaquettes sanguines sont séparées du plasma par centrifugation dans une première étape et au moins une partie des plaquettes sanguines séparées est ensuite lavée une ou plusieurs fois avec un agent de lavage approprié dans une deuxième étape afin de séparer les plaquettes sanguines des protéines plasmatiques, l'agent de lavage approprié étant de préférence choisi dans le groupe composé d'une solution saline, d'une solution saline tamponnée au phosphate (PBS), d'une solution de Ringer, d'une solution de fluide corporel simulé (SBF), de préférence une solution saline NaCl de 0,9 % en poids.

4. Procédé selon l'une des revendications 1 à 3, dans lequel les protéines plasmatiques à l'étape a) et à l'étape b) comprennent ou sont composées d'une ou de plusieurs protéines choisies dans le groupe constitué de globulines, de préférence d'immunoglobulines et de manière encore plus préférée d'anticorps contre des groupes sanguins.

5. Procédé selon l'une des revendications 1 à 4, dans lequel la procédure de lavage à l'étape c) est répétée une fois, deux fois, trois fois ou plus et dans lequel les facteurs de croissance séparés à chaque étape de séparation sont combinés de manière à former l'extrait de facteur de croissance allogénique à l'étape c).

6. Procédé selon l'une des revendications 1 à 5, dans lequel de l'eau distillée ou de l'eau déionisée, de préférence de l'eau distillée est utilisée comme eau à l'étape c).

7. Procédé selon l'une des revendications 1 à 6, dans lequel à l'étape d), l'extrait de facteur de croissance allogénique séparé est réfrigéré dans une première étape de préférence pendant une période de 8 à 24 heures à des températures entre -30°C et -20°C et est séché dans une deuxième étape pendant une période de 12 à 32 heures à des températures entre 20°C et 30°C de manière à former l'extrait de facteur de croissance allogénique lyophilisé.

8. Procédé selon l'une des revendications 1 à 7, dans lequel dans une autre étape de procédé, au moins une partie des facteurs de croissance dans l'extrait de facteur de croissance allogénique lyophilisé est déterminée et l'extrait de facteur de croissance allogénique est marqué de manière correspondante.
